(19) Europäisches Patentamt
European Patent Office
Office européen des brevets

(11) Veröffentlichungsnummer: **0 172 365**
**A1**

# (12) EUROPÄISCHE PATENTANMELDUNG

(21) Anmeldenummer: **85107974.9**

(22) Anmeldetag: **27.06.85**

(51) Int. Cl.[4]: **C 07 C 57/32**
C 07 C 51/353, C 07 C 51/09
C 07 C 69/614, C 07 C 67/36

(30) Priorität: **21.08.84 DE 3430662**

(43) Veröffentlichungstag der Anmeldung:
**26.02.86 Patentblatt 86/9**

(84) Benannte Vertragsstaaten:
**CH DE FR GB IT LI**

(71) Anmelder: **HÜLS AKTIENGESELLSCHAFT**
**- RSP Patente / PB 15 - Postfach 13 20**
**D-4370 Marl 1(DE)**

(72) Erfinder: **Hög, Hans-Ulrich, Dr.**
**Bitterfelder Strasse 9 a**
**D-4370 Marl(DE)**

(72) Erfinder: **Bub, Günther, Dr.**
**Kampstrasse 94**
**D-4370 Marl(DE)**

## (54) Verfahren zur Herstellung von Phenylessigsäure und deren Derivaten durch katalytische Umlagerung von Benzylformiat und dessen Derivaten.

(57) Zur Herstellung von Phenylessigsäure und deren Derivaten durch katalytische Umlagerung von Benzylformiat und dessen Derivaten setzt man ein Metallkatalysatorsystem aus Rhodium bzw. Rhodiumsalzen oder Rhodiumkomplexen, aus Halogen oder einer Halogenverbindung, aus einer Metallverbindung der VI. Nebengruppe, bevorzugt Chromcarbonyl, Chromhalogenide und oder Chromacetat, und/oder Liganden aus der Gruppe der organischen Stickstoff- oder Phosphorverbindungen als Promotor ein. Die Konzentration dieses Katalysatorsystems beträgt 0,05 bis 5, vorzugsweise 0, 2 bis 2 mg-atom Rhodium pro Mol eingesetztes Benzylformiat.

Die Umsetzung erfolgt in Gegenwart von Kohlenmonoxid in der Flüssig- oder Gasphase bei Temperaturen von 140 bis 300°C und CO-Partialdrücken bei diesen Reaktionstemperaturen von 2 bis 250 bar.

Verfahren zur Herstellung von Phenylessigsäure und deren Derivaten durch katalytische Umlagerung von Benzylformiat und dessen Derivaten

Die Erfindung betrifft ein Verfahren zur Herstellung von Phenylessigsäuren, insbesondere Phenylessigsäure selbst, durch Umlagerung des entsprechenden Ameisensäureesters. Die Umlagerung erfolgt in Gegenwart von Kohlenmonoxid in praktisch wasserfreiem Medium in Gegenwart eines Katalysators, bestehend aus einer Rhodiumkomponente, einer Jod- oder Bromkomponente als Promotor und einer weiteren Metallkomponente aus der VI. Nebengruppe und/oder einem Liganden aus der Gruppe der organischen Stickstoff- oder Phosphorverbindungen, anstelle oder zusätzlich zu dieser zweiten Metallkomponente.

Phenylessigsäure ist als Ausgangsmaterial für die Herstellung von Arzneimitteln, vor allem bei der Produktion von Penicillin, und von Herbiziden von industrieller Bedeutung; zahlreiche ihrer Ester finden Verwendung als Geruchs- und Geschmacksstoffe (Kirk-Othmer, Vol. 15, Seite 215, 2. Auflage 1968). In der Literatur werden zahlreiche Synthesewege für die Säure beschrieben, von denen die saure oder alkalische Hydrolyse des Benzylcyanids technische Bedeutung besitzt.

Neuere Verfahren beruhen auf der durch einen Übergangsmetallkatalysator bewirkten Carbonylierung eines Benzylalkohols, -esters oder -halogenids. Die DE-OS 22 59 072 (= GB-PS 1 410 400 = US-PS 3 928 429) beschreibt die Umsetzung von Benzylchlorid oder eines ringsubstituierten Derivats in wäßrig-alkoholischer Lösung, unter Katalyse durch Cobalt- oder Eisencarbonyl und in Gegenwart einer zum organischen Halogenid mindestens stöchiometrischen Menge einer basischen Verbindung, zu einem Produktgemisch der Säure und ihres Esters mit dem eingesetzten Alkohol. Der Katalysator dieses

Verfahrens ist schwierig zu handhaben und kann nicht aus einfachen Vorstufen, z. B. Metallsalzen, während der Reaktion gebildet werden. Nach der DE-OS 26 06 655 (= GB-PS 1 567 494) kann man die Reaktion auch mit einem Rhodiumkatalysator, dem ein Halogenpromotor zugesetzt wird, durchführen; mit einem Alkohol als Lösemittel und einer gleichfalls stöchiometrischen Menge einer Base bilden sich der entsprechende Ester als Haupt- und der Ether als Nebenprodukt. Verfahren nach diesem Prinzip laufen unter sehr milden Bedingungen ab, besitzen aber den Nachteil, daß je Mol entstandener Säure zwei und je Mol Ester ein Mol an Abfallsalzen anfallen.

Ein stöchiometrischer Zusatz einer Base ist nicht erforderlich, wenn man einen Alkohol, Ether oder Ester in die Carbonylierungsreaktion einsetzt. Unter anderem für Benzylderivate beschreibt die US-PS 3 769 326 diesen Reaktionstyp in Gegenwart eines Rhodium-, die US-PS 3 769 324 in Gegenwart eines Iridium-, Osmium- oder Rutheniumkatalysators, jeweils zusammen mit einem Halogenpromotor. Dabei beginnt die Reihe der Ester mit den Acetaten. Wasser begünstigt die Umsetzung; im Fall der Ether und Ester ist es sogar in mindestens äquimolarer Menge erforderlich, besser aber setzt man es in 50 bis 300 %igem Überschuß ein. Die Gegenwart des Wassers erschwert die Produktgewinnung und verstärkt die Korrosionswirkung des Halogenpromotors stark. Benzylhalogenide lassen sich unter Hydrolysebedingungen in gleicher Weise umsetzen, doch fällt dabei letztlich ein Mol Abfallsalz pro Mol hergestellter Säure an, wenn man die entstehende Halogenwasserstoffsäure entfernt.

Die Verwendung einer Chrom- gemeinsam mit einer Rhodiumkomponente auf einem Trägerkatalysator, wie sie in der DE-AS 19 66 695 (= US-PS 3 717 670) beschrieben wird, bezieht sich auf ein Gasphasenverfahren. Im Beispiel 27 der DE-AS wird ohne Zahlenangaben die Anwendbarkeit des Verfahrens auf die Carbonylierung von Benzylalkohol erwähnt. Der Vergleich von Beispielen mit Methanol als Ausgangsmaterial mit entsprechenden Beispielen etwa der DE-PS 17 67 151 (= US-PS 3 689 533), die in flüssiger Phase mit homogen gelöstem Katalysator und ohne Chromkomponente durchgeführt wurden, zeigt, daß die Katalysatoraktivität im Gasphasenverfahren weitaus geringer ist. Auch hier erfordern die Ausgangsmaterialien Ester, Ether und Halogenide mindestens stöchiometrische Einsatzmengen Wasser.

Unter gleichartigen Druck- und Temperaturbedingungen wie mit den Edelmetallkatalysatoren gelingt die Carbonylierung von Benzylalkohol, nicht aber die seiner Derivate, wie etwa den Estern, mit Nickelkatalysatorsystemen (EP-PSS 18 927, 39 652, 39 653, 39 654). Dabei benötigt man jedoch unvorteilhaft hohe Konzentrationen der Nickelverbindung, des Halogenpromotors und weiterer Komponenten.

Weiterhin ist die übergangsmetallkatalysierte Umlagerung von Benzylformiat bekannt. Dieser Syntheseweg ist insofern besonders vorteilhaft, weil er mehrere der vorstehend geschilderten Nachteile vermeidet. Benzylalkohol läßt sich durch Oxidation von Toluol herstellen und durch Veresterung mit Ameisensäure oder durch Carbonylierung mit Kohlenmonoxid oder Synthesegas ins Formiat überführen; bei diesem Darstellungsweg fallen Abfallsalze nicht an. Bei der darauffolgenden Umlagerung zur Phenylessigsäure lassen sich die mit einem Wassereinsatz verbundenen Probleme vermeiden.

Verfahren, die Katalysatorsysteme aus Nichtedelmetallkatalysatoren, Halogenpromotoren und gegebenenfalls weiteren Zusätzen sowie speziellen Lösemitteln anwenden, wie die DE-PS 10 72 979 und die DE-PS 20 26 031 (= US-PS 3 839 428), liefern selbst bei gleichzeitig hohen Temperaturen und Drücken von 200 $^{o}$C und 200 bar und darüber nur niedrige Ausbeuten, bezogen auf die eingesetzte Katalysatormenge.

Die Anwendung von Edelmetallkatalysatoren bei milderen Bedingungen für die Umlagerung von Formiaten der Form HCOOR mit R = $C_2$ und höher ist ebenfalls beschrieben. Das in der JP-OS 56-22745 beschriebene System aus einer Palladium- und Halogenverbindung sowie einem Amin, Amid oder Phosphan zeigt nur eine unbefriedigende Aktivität und Selektivität. Mit einem Katalysator aus einer Iridium- und einer Jodverbindung erfolgt nach der EP-PS 45 637 die Umlagerung des Ameisensäureesters ohne Anwendung eines Kohlenmonoxiddrucks. Aus den Heißdrücken und den Massenbilanzen der angeführten Beispiele folgt jedoch, daß Kohlenmonoxid durch Zersetzung des Formiats erzeugt wird. Gekennzeichnet ist das Verfahren außerdem dadurch, daß die Gegenwart einer Carbonsäure als Lösemittel unbedingt erforderlich ist. Katalysatoren auf Basis von Rhodium werden als ungeeignet bezeichnet. Aus beiden Schriften geht das spezielle Verhalten von Benzylformiat oder ringsubstituierten Derivaten nicht hervor.

Für die Umlagerung von Methylformiat zu Essigsäure gibt es in der Literatur eine Anzahl weiterer Beschreibungen. Für die vorliegende Erfindung von einer gewissen Bedeutung sind die DE-OS 30 46 899, die DE-PS 21 09 025 (= US-PS 3 798 267), die US-PS 4 194 056, die DE-OS 32 36 351 (= EP-OS 105 132) und die unveröffentlichte DE-Anmeldung 33 33 317.3.

Diese Veröffentlichungen beschreiben Katalysatorsysteme aus einer Palladium-, Iridium-, Ruthenium- oder Rhodiumkomponente, einem Halogenpromotor und einer organischen Verbindung eines Elementes der V. Hauptgruppe oder einer zweiten Metallkomponente unter anderem aus der VI. Nebengruppe, jedoch ausschließlich für die Umlagerung von Methylformiat zu Essigsäure. Die Sonderstellung, die Methylformiat in bezug auf seine Reaktivität gegenüber den höheren Ameisensäureestern vor allem bei technisch erwünschten, vergleichsweise milden Reaktionsbedingungen von nicht mehr als 200 °C und 50 bar Heißdruck besitzt, geht aus der Literatur hervor. Anschaulich zeigen die nachfolgenden Vergleichsbeispiele a und b das Ausmaß der Verschlechterung der Reaktivität eines gegebenen Katalysatorsystems, wenn man anstelle des Methylesters den Ethylester umlagert.

Hieraus ergab sich die Aufgabe, ein einfaches Verfahren zur Herstellung von Phenylessigsäure oder deren Derivaten zu finden.

Diese Aufgabe wird erfindungsgemäß entsprechend den Angaben der Patentansprüche gelöst.

Es ist überraschend und aus dem Stand der Technik in keiner Weise abzuleiten, daß die Umlagerung von Benzylformiat oder deren Derivaten zu Phenylessigsäure oder deren Derivaten unter gleichartigen, relativ milden Reaktionsbedingungen und ähnlich glatt wie im Fall des Methylformiats gelingt, indem man die nachstehend näher beschriebenen Katalysatorsysteme und Reaktionsbedingungen anwendet. In der Verfahrensbeschreibung werden die Begriffe "Benzylformiat" und "Phenylessigsäure" stellvertretend auch für deren Derivate verwendet. Weiterhin ist mit dem Begriff "Umlagerung" keine Einschränkung des Verfahrens auf einen bestimmten, tatsächlich nicht näher bekannten Reaktionsablauf verbunden.

Im erfindungsgemäßen Verfahren bringt man Benzylformiat hinreichend lange, im allgemeinen 0,1 bis 10 Stunden, bei einer erhöhten Temperatur von 140 bis 300 °C in Gegenwart von Kohlenmonoxid bei einem CO-Partialdruck bei Reaktionstemperatur von 2 bis 250 bar mit einem Katalysatorsystem in Kontakt, welches Rhodium, ein Rhodiumsalz oder einen Rhodiumkomplex, ein Halogen als Promotor und eine zweite Metallkomponente in Form einer Verbindung eines Elementes der VI. Nebengruppe oder statt dessen bzw. zusätzlich einen Liganden aus der Gruppe der organischen Stickstoff- und Phosphorverbindungen enthält. Die Reaktion erfolgt in weitgehend wasserfreiem Medium.

Rhodium kann elementar in fein verteilter Form, als anorganisches oder organisches Salz oder als Komplexverbindung eingesetzt werden. Dabei ist es vorteilhaft, daß sich aus so einfachen Verbindungen wie $RhCl_3 \cdot XH_2O$, die eine ausgezeichnete Wirksamkeit für das Verfahren zeigen, unter Reaktionsbedingungen die aktive Katalysatorform ohne spezielle Präformierung schnell bildet. Weitere geeignete Einbringungsformen sind $Rh_2O_3$, $RhBr_3$, $RhJ_3$, Rhodium-(III)-acetylacetonat, Rhodium-(II)-acetat-Dimer, Chloro-dicarbonylrhodium-(I)-Dimer, Tetrarhodiumdodecacarbonyl und andere mehr. Geeignet sind auch Rhodiumkomplexe, die schon einen Stickstoff- oder Phosphorliganden enthalten, wie beispielsweise $Rh(Pyridin)_3Cl_3$, $RhCl(PPh_3)_3$, $RhCl(CO)(PPh_3)_2$ und andere mehr.

Als Halogenpromotoren eignen sich Jod und, mit schwächerer Promotorwirkung, Brom als Element oder in Form einer Verbindung. Geeignete Einsatzformen umfassen Verbindungen der Form $X_3^-$ und HX mit X = J oder Br, anorganische und organische Jodide und Bromide. Als anorganische Verbindungen können Salze derjenigen Metalle dienen, die die Reaktion ihrerseits nicht ungünstig beeinflussen, wie zum Beispiel Jodide und Bromide der Alkali- und Erdalkalimetalle oder von den Übergangsmetallen die des Nickels oder auch der Katalysatormetalle selbst, als organische Verbindungen Alkyl-, Acyl- und Aryljodide und -bromide. Diese Verbindungen können einzeln oder im Gemisch miteinander eingesetzt werden. Geeignet sind auch Addukte einer organischen Jod- oder Bromverbindung mit dem Stickstoff- oder Phosphorliganden, wie beispielsweise ein Tetraalkylammonium- oder -phosphoniumjodid oder ein N-Alkylpyridiumjodid. Um die erwünschten hohen Phenylessigsäureausbeuten zu erhalten, sollte jedoch nicht die gesamte Menge an Promotor in dieser gebundenen, sondern ein Teil noch in freier Form vorliegen.

Entsprechend sollten Promotor und Stickstoff- oder Phosphorverbindung in solchen Mengen eingesetzt werden, daß das Verhältnis von gesamter Menge an Jod- oder Bromatomen zur gesamten Menge an Stickstoff- oder Phosphoratomen größer als 1 ist. Bevorzugt sind Jod und seine Verbindungen, insbesondere Methyljodid und Benzyljodid.

Geeignete organische Stickstoffverbindungen gehören zu den Gruppen

(a) der heterocyclischen aromatischen Amine, wie beispielsweise Chinolin, Isochinolin und deren Derivate, Pyridin und dessen substituierte Derivate wie die Picoline und Lutidine;

(b) der aromatischen Amine, wie beispielsweise die N,N-Dialkylaniline, N-Alkyldiphenylamine oder am aromatischen Ring substituierte Derivate derselben;

(c) der aliphatischen, cycloaliphatischen und araliphatischen Amine, wie beispielsweise Tri-n-alkylamine wie Triethyl- bis Tri-n-dodecylamin oder entsprechende Verbindungen mit ungleichen Alkylgruppen, verzweigte Alkylamine wie das Tris-(2-ethylhexyl)-amin oder das Tricyclohexylamin, N-Alkylpyrrolidine und -piperidine oder N-Alkylmorpholine, weiterhin die entsprechenden primären und sekundären Amine;

(d) Carbonsäureamide, wie Form-, Acet- oder Phenylacetamid und ihre N-Alkyl- und N,N-Dialkylderivate sowie entsprechende Verbindungen höherer Carbonsäuren, zu denen beispielsweise auch aromatische Amide wie das Benzamid, N-Alkyl- und N,N-Dialkylbenzamide, sowie Lactame wie Pyrrolidon, N-Alkylpyrrolidone, Piperidon und N-Alkylpiperidone, gehören.

Bevorzugte organische Stickstoffverbindungen sind die heterocyclischen aromatischen Amine der Gruppe (a), die N,N-disubstituierten Aniline der Gruppe (b), die mit kurzen ($C_1$- bis $C_4$-)Alkylresten N-substituierten cycloaliphatischen Monoamine der Gruppe (c) und die N,N-disubstituierten Carbonsäureamide der Gruppe (d). Bei den Verbindungen der Gruppe (d) sind die Form- und die Phenylacetamide besonders bevorzugt, da mit ihnen dieselben Carbonsäurereste eingeführt werden, die schon zum Reaktionsgemisch gehören.

Geeignete organische Phosphorverbindungen sind tertiäre Phosphane der allgemeinen Form $R_3P$ und $R_2P(CH_2)_nPR_2$, wobei die Reste R gleiche oder verschiedene Alkylgruppen mit 1 bis 10 C-Atomen oder Phenyl-, substituierte Phenyl-, Phenylalkyl- oder Naphthylgruppen bedeuten und n = 1 bis 6 beträgt. Bei aliphatischen Resten R kann es sich um unverzweigte, verzweigte oder cyclische Substituenten handeln; beide Reste R an einem Phosphoratom können mit diesem auch einen Ring bilden. Bevorzugte Phosphorverbindung ist das Triphenylphosphan.

Bevorzugter Zusatz zum Rhodium-Halogen-Katalysator ist eine Verbindung eines Metalls der VI. Nebengruppe. Von diesen sind Chrom- und Molybdänverbindungen bevorzugt, insbesondere Chromverbindungen. Geeignete Einsatzformen umfassen die Carbonyle, Halogenide, Acetate, Acetylacetonate sowie andere organische oder anorganische Salze und Komplexe der Metalle, wobei sich die Bedingung von selbst versteht, daß derartige Verbindungen keine den Rhodiumkatalysator vergiftenden Bestandteile enthalten dürfen.

Als Ausgangsverbindungen für die Umlagerung eignen sich Benzylformiate der allgemeinen Form I

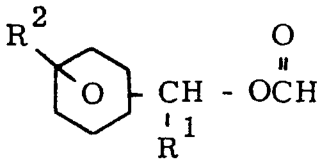

I

Darin steht $R^1$ für H oder einen n-Alkylrest mit 1 bis 6 C-Atomen und $R^2$ für H oder (a) einen beliebigen Alkylrest mit 1 bis 10 C-Atomen, (b) einen Alkoxirest $R^3O$-, wobei $R^3$ einen beliebigen Alkylrest mit 1 bis 10 C-Atomen oder eine Gruppe $R^4(OCH_2CH_2)_n$- mit $R^4$ = $C_1$-$C_4$-Alkyl und n = 1 bis 3 bedeutet, (c) eine Hydroxylgruppe, (d) eine Estergruppe $R^5COO$- mit $R^5$ = $C_1$- bis $C_4$-Alkyl oder (e) ein Halogenatom. Der Einsatz von Verbindungen mit Substituenten $R^2$ gemäß (c) oder (d) gibt durch Selbstveresterung bzw. Umesterung teilweise Oligomere. Besonders glatt verläuft die Reaktion, wenn $R^1$ = H ist. Die vorliegende Erfindung betrifft insbesondere die Umlagerung des Benzylformiats selbst.

Die Umsetzung erfolgt bevorzugt in der Flüssigphase. Die Verwendung eines Lösemittels ist nicht unbedingt erforderlich, da die Reaktion im flüssigen oder geschmolzenen Ausgangsmaterial bzw. Produkt glatt abläuft. Falls aus technischen Gründen die Verwendung eines Lösemittels erwünscht sein sollte, können solche ohne weiteres angewendet werden. Beispiele dafür sind andere Carbonsäuren, Ketone und Ether, wobei die Auswahl einer speziellen Verbindung selbstverständlich abhängt von der Möglichkeit zur späteren Abtrennung vom Produkt. Alkohole oder Ester sind als Lösemittel deshalb unzweckmäßig, weil sie durch Umesterung mit dem Benzylformiat und eventuell durch Folgereaktionen das Produktgemisch komplizieren und die erzielbare Ausbeute an Phenylessigsäure vermindern. Ist allerdings eine Produktlösung in Essigsäure erwünscht, kann ohne weiteres Methylformiat oder Methanol als Lösemittel für den Einsatz benutzt werden, aus denen sich dann gleichzeitig mit dem Produkt die Essigsäure bildet.

Es ist vorteilhaft, die Umsetzung in praktisch wasserfreiem Medium durchzuführen, um auf einfache Weise absolutes Produkt zu erhalten, die Korrosivität des Mediums zu vermindern und unerwünschte Nebenreaktionen, die in Gegenwart größerer Wassermengen deutlich zunehmen, zu vermeiden. Geringe Wassergehalte von nicht mehr als 5 Gewichtsprozent, günstiger von nicht mehr als 2 Gewichtsprozent, können jedoch geduldet werden. Auch stören Verunreinigungen mit Benzylalkohol und -acetat, Benzaldehyd und seinem Diacetat oder Benzoesäure und ihr Benzylester, wie sie in technischem Benzylalkohol enthalten sein können, nicht.

Die Katalysatorkomponenten werden in der Flüssigphase suspendiert oder homogen gelöst, wobei die Metallkomponenten auch auf Trägern, wie Aktivkohle, Aluminiumoxid oder Kieselgel, aufgebracht sein können. Das Verfahren kann auch in der Gasphase durchgeführt werden, wobei die metallhaltigen Katalysatorbestandteile in fester Form oder auf einen Träger, wie Aktivkohle, Kieselgel oder Aluminiumoxid, aufgebracht mit einem gasförmigen Einsatzgemisch aus dem Edukt, einer flüchtigen Jod- oder Bromverbindung, gegebenenfalls einem Liganden und Kohlenmonoxid in Kontakt gebracht werden. Bei hinreichender Schwerflüchtigkeit bringt man den Liganden ebenfalls auf den Träger auf bzw. löst oder suspendiert die metallhaltigen Komponenten in der Schmelze. Bevorzugt ist aber die Durchführung in der Flüssigphase, die bessere Umsätze und/oder Selektivitäten gibt. Das Verfahren läßt sich kontinuierlich oder diskontinuierlich durchführen.

Nach beendeter Umsetzung kann das Produkt destillativ abgetrennt werden; flüchtige Jod- bzw. Bromverbindungen werden dabei als Vorlauf abgenommen und gemeinsam mit dem bei Durchführung in der Flüssigphase den Katalysator enthaltenden Destillationssumpf in den Reaktor zurückgeführt. Je nach Art der verwendeten Liganden werden diese mit dem Katalysator oder als eine Destillationsfraktion ebenfalls in den Prozeß zurückgeführt. Das erfindungsgemäße Verfahren ist jedoch auf eine bestimmte Ausprägung der technischen Ausführung nicht beschränkt.

Die Reaktion erfolgt in Gegenwart von Kohlenmonoxid. Dieses wird zwar gemäß der Stöchiometrie für die Umsetzung nicht benötigt, dient aber zur Stabilisierung des Ameisensäureesters und der aktiven Katalysatorform gegen Zersetzung und nötigenfalls zur Bildung dieser aktiven Katalysatorform aus den eingesetzten Verbindungen. Das Gas kann, gegebenenfalls unter Abzweigen eines kleinen Abfallstroms zum Ausschleusen gasförmiger Verunreinigungen, im Kreis geführt werden. Verunreinigungen des Kohlenmonoxids durch Wasserstoff, Stickstoff, Methan, Kohlendioxid oder andere inerte Gase stören auch in größerer Menge nicht; das Verfahren kann sogar unter Synthesegasdruck durchgeführt werden. Fremdgase sollten aber gering gehalten werden, um zur Einstellung des notwendigen CO-Partialdrucks keinen unerwünscht hohen Reaktionsdruck zu benötigen. Bei Verwendung von reinem Kohlenmonoxid wird das Verfahren bei CO-Drücken von 2 bis 250 bar, bevorzugt bei 10 bis 100 bar, durchgeführt. Typische CO-Kaltdrücke im bevorzugten Bereich bei einer Durchführung der Reaktion in der Flüssigphase sind 5 bis 80 bar. Der zur Stabilisierung des Eduktes oder des Katalysators vorzulegende Mindestdruck an Kohlenmonoxid ist von der Reaktionstemperatur abhängig und kann bei Bedarf schnell und einfach ermittelt werden.

Die Umsetzungstemperatur des Verfahrens beträgt 140 bis 300 °C, bevorzugt 160 bis 220 °C. Die Reaktionsdauer kann in Abhängigkeit von den übrigen Verfahrensparametern in weiten Grenzen variieren und wird zweckmäßigerweise so eingerichtet, daß ein praktisch vollständiger Umsatz erreicht wird, im allgemeinen in 0,1 bis 10 Stunden.

Eine Reaktionsdauer von zwei Stunden und weniger läßt sich dabei ohne weiteres erreichen.

Es ist leicht einzusehen, daß der Einsatz einer möglichst geringen Menge an Katalysator, vor allem des Rhodiums als dem kostspieligsten Bestandteil, wirtschaftlich wünschenswert ist. Daher ist es von Vorteil, daß das Verfahren im Vergleich zu Katalysatorsystemen, die auf anderen Metallen aufbauen, mit sehr geringem Katalysatoreinsatz auskommt. Geeignet sind Mengen an Rhodium(verbindung), die 0,05 bis 5 mg-atom Metall pro Mol Ameisensäurebenzylester, bevorzugt 0,2 bis 2 mg-atom/Mol, entsprechen. Dabei beträgt das Atomverhältnis Rhodium zu Halogen, bevorzugt Rh/J, 1 : 1 000 bis 1 : 1, bevorzugt 1 : 100 bis 1 : 5. Verwendet man eine als zusätzliche Komponente bevorzugte Verbindung der VI. Nebengruppe, so beträgt das Atomverhältnis zu Rhodium, bevorzugt Rh/Cr, 1 : 100 bis 10 : 1, vorzugsweise 1 : 20 bis 2 : 1. Setzt man einen der als zusätzliche Komponente weniger bevorzugten Liganden aus der Gruppe der organischen Stickstoff- oder Phosphorverbindungen ein, so beträgt das Verhältnis zum Rhodium, ausgedrückt als Atomverhältnis Rh/N bzw. Rh/P, 1 : 100 bis 1 : 1, bevorzugt 1 : 40 bis 1 : 2, wobei es nicht erforderlich ist, die organische Stickstoffverbindung als Lösemittel in Mengen von mindestens 0,2 Mol/Mol Ester einzusetzen. Es wird vorzugsweise ein Verhältnis des J oder Br zu N bzw. P von größer als 1 eingehalten, um besonders gute Ergebnisse zu erzielen. Die drei Typen zusätzlicher Komponenten können sowohl einzeln als auch im Gemisch miteinander eingesetzt werden. Man kann zwar grundsätzlich auch größere Mengen jeder Komponente anwenden, jedoch bewirkt das keine Verbesserung und ist wirtschaftlich ungünstig. Bei kleineren Mengen dagegen ist es notwendig, in unerwünschter Weise die Umsetzungsdauer zu verlängern oder die Reaktionsbedingungen zu verschärfen.

Durch die folgenden Beispiele wird das Verfahren weiter erläutert.

Beispiel 1

In einem 100-ml-Autoklaven aus Hastelloy C werden 46,4 g eines 93prozentigen Benzylformiats (317 mMol), 0,1 g $RhCl_3 \cdot 3H_2O$ (0,38 mMol), 0,4 g $Cr(O)_6$ (1,8 mMol) und 2,5 g Methyljodid (17,6 mMol) vorgelegt. Der Autoklav wird druckfest verschlossen und mit Kohlenmonoxid gespült. Anschließend wird Kohlenmonoxid bis 30 bar aufgepreßt, auf 180 °C aufgeheizt, der Reaktionsdruck mittels geringer CO-Zugabe auf 50 bar eingestellt und die Umsetzung unter intensivem Rühren 2 Stunden lang durchgeführt. Danach erfolgt schnelles Abkühlen (15 Minuten) und Entspannen. Nach Zusatz einer definierten Menge 1,4-Dioxan als innerem Standard wird der Autoklav entleert und das Produkt gaschromatographisch analysiert. Der Umsatz des Benzylformiats beträgt 97 %; es finden sich die aufgeführten Produkte mit den folgenden Selektivitäten, bezogen auf die Zahl der Benzylgruppen:

| | |
|---|---|
| Phenylessigsäure | 78,5 % |
| Phenylessigsäurebenzylester | 16 % |
| Benzylalkohol | $<$ 0,1 % |
| Benzylacetat | 0,8 % |
| (durch Reaktion mit CO und $CH_3J$) | |
| Benzaldehyd | 0,7 % |
| Toluol | 4 % |

Vergleichsbeispiel a

Die Reaktion des Beispiels 1 wird wiederholt, jedoch mit 41 g Methylformiat (682 mMol) als Ausgangsmaterial und der doppelten Menge aller drei Katalysatorkomponenten. Nach der gleichen Zeit von 2 Stunden beträgt der Umsatz 99,8 % und die Selektivität für Essigsäure ebenfalls 99,8 %. Man erkennt, daß Benzylformiat und Methylformiat ähnlich rasch umgelagert werden.

Vergleichsbeispiel b

Vergleichsbeispiel a wird wiederholt mit 50,5 g Ethylformiat (92prozentig) (628 mMol). Bei 180 °C beobachtet man keinen Umsatz innerhalb von 2 Stunden. Bei 210 °C werden in der gleichen Zeit 38,5 % umgesetzt. Propionsäure und Propionsäureethylester entstehen im Selektivitätsverhältnis von 4 : 3. Dieser Versuch zeigt, daß ein anderes Formiat als das Benzylformiat deutlich schlechter reagiert als das Methylformiat.

Vergleichsbeispiel c

Beispiel 1 wird wiederholt, wobei man statt des Rhodiumkatalysators eine äquivalente Menge $IrCl_3\ 3\,H_2O$ einsetzt. Während der Reaktion steigt der Druck im Autoklaven bis auf 75 bar. Die Analyse des Austrags zeigt, daß das Benzylformiat vollständig umgesetzt ist. Das Produktgemisch enthält:

| | |
|---|---|
| Phenylessigsäure | 50 mMol |
| Phenylessigsäuremethylester (durch Reaktion mit $CH_3J$) | 1 mMol |
| Benzaldehyd | 7 mMol |
| Toluol | 10 mMol |
| Ameisensäure | 63 mMol |

Die präparative Aufarbeitung des Gemisches durch alkalische Extraktion, Ansäuern der abgetrennten Phase und Rückextraktion mit Diethylether gibt eine Säurefraktion, die neben rund 50 Gewichtsprozent Phenylessig- und Ameisensäure weitere, unbekannte Komponenten enthält. Daneben fällt eine zähflüssige Neutralfunktion in vergleichbarer Gewichtsmenge an. Dieses Vergleichsbeispiel zeigt, daß ein Katalysatorsystem auf Basis einer Iridiumkomponente sich für die Umlagerung von Benzylformiat nicht eignet.

Patentansprüche:

1. Verfahren zur Herstellung von Phenylessigsäure und deren Derivaten durch katalytische Umlagerung von Benzylformiat der allgemeinen Formel I

I 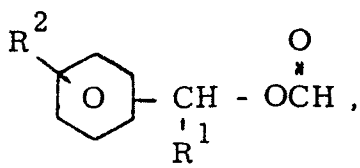

in der $R^1$ für H oder einen n-Alkylrest mit 1 bis 6 C-Atomen und $R^2$ für H oder (a) einen beliebigen Alkylrest mit 1 bis 10 C-Atomen, (b) einen Alkoxirest $R^3O$-, wobei $R^3$ einen beliebigen Alkylrest mit 1 bis 10 C-Atomen oder eine Gruppe $R^4$-$(OCHCH_2)_n$- mit $R^4$ = $C_1$- bis $C_4$-Alkyl und n = 1 bis 3 bedeutet, (c) eine Hydroxylgruppe, (d) eine Estergruppe $R^5COO$- mit $R^5$ = $C_1$- bis $C_4$-Alkyl oder (e) ein Halogenatom steht, in Gegenwart eines Metallkatalysators, eines Halogenpromotors und von Kohlenmonoxid bei erhöhter Temperatur und erhöhtem Druck,

dadurch gekennzeichnet,

daß man die Umsetzung in der Flüssigphase an einem Metallkatalysatorsystem aus Rhodium bzw. Rhodiumsalzen oder Rhodiumkomplexen, einem Halogen oder einer Halogenverbindung als Promotor, einem Salz oder einem Komplex eines Elementes der VI. Nebengruppe und/oder Liganden aus der Gruppe der organischen Stickstoff- oder Phosphorverbindungen bei Temperaturen von 140 bis 300 °C und CO-Partialdrücken bei diesen Reaktionstemperaturen von 2 bis 250 bar durchführt, wobei man das Rhodium oder die Rhodiumverbindung in Mengen von 0,05 bis 5 mg-atom Rhodium pro Mol eingesetztes Benzylformiat einsetzt und ein Atomverhältnis Rhodium : Halogen von 1 : 1 000 bis 1 : 1,

ein Atomverhältnis Rhodium : Metall der VI. Nebengruppe

von 1 : 100 bis 10 : 1 sowie gegebenenfalls ein Atomverhältnis Rh : N oder P von 1 : 100 bis 1 : 1 einhält.

2. Verfahren nach Anspruch 1, dadurch gekennzeichnet, daß man als Halogenpromotoren Jod, Brom oder Verbindungen von Jod und/oder Brom, bevorzugt Methyljodid und Benzyljodid einsetzt.

3. Verfahren nach den Ansprüchen 1 und 2, dadurch gekennzeichnet, daß man als Metallverbindung der VI. Nebengruppe Chrom- und/oder Molybdänverbindungen, vorzugsweise Chromverbindungen, wie Chromcarbonyl, Chromhalogenid und/oder Chromacetat, einsetzt.

4. Verfahren nach den Ansprüchen 1 und 2, dadurch gekennzeichnet, daß man als organische Stickstoffverbindung heterocyclische aromatische, aromatische, aliphatische, cycloaliphatische und araliphatische Amine und/oder Carbonsäureamide einsetzt, bevorzugt heterocyclische aromatische Amine, N,N-disubstituierte Aniline und N-substituierte cycloaliphatische Monoamine, insbesondere mit $C_1$- bis $C_4$-Substituenten am N-Atom, sowie N,N-disubstituierte Carbonsäureamide, insbesondere Formamide und Phenylacetamide.

5. Verfahren nach den Ansprüchen 1 und 2, dadurch gekennzeichnet, daß man als organische Phosphorverbindung Triphenylphosphan einsetzt.

6. Verfahren nach den Ansprüchen 1 bis 5, dadurch gekennzeichnet, daß man das Rhodium bzw. die Rhodiumverbindung in Mengen von 0,2 bis 2 mg-atom Rhodium pro Mol eingesetztes Benzylformiat einsetzt.

7. Verfahren nach den Ansprüchen 1 bis 6, dadurch gekennzeichnet, daß man in dem Metallkatalysatorsystem ein Atomverhältnis Rhodium : Halogen von 1 : 100 bis 1 : 5, Rhodium : Metall der VI. Nebengruppe von 1 : 20 bis 2 : 1 und gegebenenfalls Rh : N oder P von 1 : 40 bis 1 : 2, einhält.

8. Verfahren nach den Ansprüchen 1, 2, 4 und 5 dadurch gekennzeichnet, daß man ein Verhältnis Halogen : N oder P von größer als 1 einhält.

9. Verfahren nach den Ansprüchen 1 bis 8, dadurch gekennzeichnet, daß man als Benzylformiat der allgemeinen Formel I Benzylformiat einsetzt.

10. Verfahren nach den Ansprüchen 1 bis 9, dadurch gekennzeichnet, daß man die Umsetzung bei einer Temperatur von 160 bis 220 °C und Reaktionsdrücken von 10 bis 100 bar durchführt.

11. Katalysatorsystem zur Herstellung von Phenylessigsäure und deren Derivaten durch Umlagerung von Benzylformiat nach den Ansprüchen 1 bis 10,
dadurch gekennzeichnet,
daß das Katalysatorsystem aus Rhodium bzw. einer Rhodiumverbindung, einem Halogen oder einer Halogenverbindung als Promotor, aus einem Salz oder Komplex eines Elementes der VI. Nebengruppe und/oder aus Liganden aus der Gruppe der organischen Stickstoff- oder Phosphorverbindungen besteht, wobei das Atomverhältnis des Rhodiums : Halogen 1 : 1 000 bis 1 : 1, das des Rhodiums : Metall der VI. Nebengruppe 1 : 100 bis 10 : 1 und das des Rh : N oder P 1 : 100 bis 1 : 1 beträgt.

0172365

Europäisches Patentamt

# EUROPÄISCHER RECHERCHENBERICHT

Nummer der Anmeldung

EP 85 10 7974

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | GB-A-1 326 014 (JOHNSON, MATTHEY & CO.)<br>* Ansprüche 1-9 *<br>----- | 1,2,5, 11 | C 07 C 57/32<br>C 07 C 51/353<br>C 07 C 51/09<br>C 07 C 69/614<br>C 07 C 67/36 |
| | | | **RECHERCHIERTE SACHGEBIETE (Int Cl 4)** |
| | | | C 07 C 51/00<br>C 07 C 57/00<br>C 07 C 67/00<br>C 07 C 69/00 |

Der vorliegende Recherchenbericht wurde für alle Patentansprüche erstellt

| Recherchenort | Abschlußdatum der Recherche | Prüfer |
|---|---|---|
| DEN HAAG | 15-11-1985 | KLAG M.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument